**Europäisches Patentamt**

**European Patent Office**

**Office européen des brevets**

(19)

(11) Veröffentlichungsnummer : **0 341 707 B1**

(12) **EUROPÄISCHE PATENTSCHRIFT**

(45) Veröffentlichungstag der Patentschrift :
**02.09.92 Patentblatt 92/36**

(51) Int. Cl.$^5$ : **C07C 69/618,** A61K 7/46,
**C07C 67/08, C07C 67/307,**
**C07C 67/343, C07C 51/377,**
**// C07C57/42, C07C69/738**

(21) Anmeldenummer : **89108475.8**

(22) Anmeldetag : **11.05.89**

(54) **o-Methylzimtsäurephenylethylester, seine Herstellung und Verwendung als Riechstoff.**

(30) Priorität : **13.05.88 DE 3816452**

(43) Veröffentlichungstag der Anmeldung :
**15.11.89 Patentblatt 89/46**

(45) Bekanntmachung des Hinweises auf die
Patenterteilung :
**02.09.92 Patentblatt 92/36**

(84) Benannte Vertragsstaaten :
**CH DE FR GB LI NL**

(56) Entgegenhaltungen :
**Keine Entgegenhaltungen.**

(73) Patentinhaber : **Consortium für
elektrochemische Industrie GmbH
Zielstattstrasse 20
W-8000 München 70 (DE)**

(72) Erfinder : **Voss, Erdmuthe
Zielstattstrasse 109
W-8000 München 70 (DE)**
Erfinder : **Moschinsky, Werner
Sendlinger Strasse 66
W-8000 München 2 (DE)**
Erfinder : **Hofmann-Moritz, Doris
Reuterstrasse 4
W-8263 Burghausen (DE)**

**Beschreibung**

Die Erfindung betrifft o-Methylzimtsäurephenylethylester, systematische Bezeichnung 3-(2-Methylphenyl)-2-propensäure(2-phenylethyl)ester, seine Herstellung und Verwendung als Riechstoff.

Zimtsäureester finden, wie auch Zimtaldehyde oder -alkohole, eine breite Anwendung in der Parfümerie, aber auch in der Aromen- und Geschmackstoffindustrie. Sie werden zur Formulierung, Imitierung oder Fixierung blumiger Parfümkompositionen eingesetzt, wie z. B. bei der Komposition von Maiglöckchen-, Rosen-, Flieder-, Gardenia- oder Hyazinthendüften (vgl. S. Arctander: Perfume and Flavor Chemicals, Montclair, N.J. (USA), 1969).

Bei der Imitierung natürlicher Duftkörper ist es selbstverständlich erklärtes Ziel der Parfümeure, diese dem natürlichen Vorbild so täuschend ähnlich wie möglich nachzuempfinden. Diese Aufgabe wird erschwert, wenn die entsprechenden etherischen Öle nicht unzersetzt gewinnbar oder sehr teuer sind, so daß hier synthetische, naturidentische oder auch nicht naturidentische Substitute ein nützliches Werkzeug für den Pafümeur darstellen. Darüber hinaus lassen sich aber auch bei Phantasiekompositionen mit solchen Substituten ungeahnte Effekte erzielen.

Gegenstand der Erfindung ist o-Methylzimtsäurephenylethylester.

Es wurde überraschend gefunden, daß durch Einführung eines o-Methylsubstituenten an der Phenylgruppe der Säurekomponente des Zimtsäurephenylethylesters ein Riechstoff erhalten wird, der im Gegensatz zu den bisher gebräuchlichen Zimtsäureestern die schwer-blumigen Akzente in blütigen und Phantasieparfümkompositionen wesentlich zu steigern und den natürlichen Blütenduft mit exaltierender Duftfülle zu erhöhen vermag.

Ein Verfahren zur Herstellung von o-Methylzimtsäurephenylethylester ist dadurch gekennzeichnet, daß Acetessigester in einer phasentransferkatalysierten Alkylierung mit o-Methylbenzylchlorid umgesetzt wird.

Weitere bevorzugte Ausgestaltungen dieses Verfahrens sind die Halogenierung des erhaltenen 2-(o-Methylbenzyl)acetessigsäureethylesters, Umsetzung des erhaltenen 2-Halogen-2-(o-methylbenzyl)acetessigsäureethylesters mit Basen sowie Veresterung der erhaltenen o-Methylzimtsäure mit 2-Phenylethanol.

Das beschriebene Verfahren geht von den großtechnisch verfügbaren Basischemikalien Acetessigester und o-Methylbenzylchlorid aus, wobei auch die weiteren Reaktionskomponenten handelsübliche Produkte sind, weshalb eine genauere Angabe von Herstellungsverfahren unnötig erscheint.

Die phasentransferkatalysierte Alkylierung von Acetessigester mit o-Methylbenzylchlorid ist sowohl mit quartären Ammoniumsalzen, wie beispielsweise Tetrabutylammoniumbromid, wie auch mit Kronenethern, wie beispielsweise 18-Krone-6, durchführbar. Als Basen können dabei sowohl Alkalimetallhydroxide, vorzugsweise Natriumhydroxid, wie auch Alkalimetallcarbonate, vorzugsweise Pottasche, eingesetzt werden. Die Umsetzung wird bei erhöhter Temperatur, bei 70 bis 90 °C durchgeführt.

Zur Halogenierung des erhaltenen 2-(o-Methylbenzyl)acetessigsäureethylesters eignen sich vorzugsweise die elementaren Halogene oder andere Halogenierungsmittel, insbesondere Sulfurylchlorid. Der bevorzugte Temperaturbereich für diese Umsetzung liegt zwischen 25 bis 60 °C insbesondere 40 bis 55 °C.

Der Abbau des 2-Halogen-2-(o-Methylbenzyl)acetessigsäureethylester zur o-Methylzimtsäure geschieht vorzugsweise durch Einwirkung von Basen, wie Kaliumhydroxid oder Natriumhydroxid. Die so erhaltene Säure wird vorzugsweise durch azeotrope Veresterung in den Phenylethylester überführt.

o-Methylzimtsäurephenylethylester findet Verwendung als Riechstoff. Die erfindungsgemäße Verbindung zeichnet sich durch einen kräftigen, blumigen, warmen, schweren Blütenduft mit Richtung Akazie, Narzisse, Tuberose aus.

Gibt man zu linearen Blütenkompositionen - wie beispielsweise Flieder und Gardenia - anstelle herkömmlicher Zimtsäureester, wie z. B. Phenylethylcinnamat, Linalylcinnamat o.a., 10 % der erfindungsgemäßen Verbindung, so wird der blütige An-, Mittel- und besonders der Nachgeruch der Kompositionen an natürlicher Duftfülle wesentlich bereichert und verstärkt. Die Intensität und Wärme der blütigen Kompositionen gewinnen. Durch den Zusatz der erfindungsgemäßen Verbindung werden die blumigen Akzente mit natürlicher, exaltierender Fülle verstärkt. Die Duftintensität, die ausgezeichnete Haftung und das brillante Fixierungsvermögen zeichnen die erfindungsgemäße Verbindung gegenüber anderen Zimtsäureestern besonders aus.

Beispiel 1

2-(o-Methylbenzyl)acetessigsäureethylester

Zu einer auf Rückfluß erhitzten Lösung aus 455 g (3,5 mol) Acetessigester, 35 g (0,11 mol) Tetrabutylammoniumbromid und 1,2 l Toluol wurden unter gutem Rühren gleichzeitig aus zwei Tropftrichtern jeweils 344 g (2,45 mol) o-Methylbenzylchlorid und 3,5 mol 25 %-ige, wäßrige NaOH-Lösung innerhalb von 2 h zugetropft.

Anschließend rührte man noch 2 h unter Rückfluß. Nach Abkühlen der Reaktionslösung versetzte man mit Wasser, trennte die Phasen und wusch die organische Phase je einmal mit Wasser, verdünnter Phosphorsäure, nochmals mit Wasser und rührte anschließend mit festem Natriumhydrogencarbonat. Man filtrierte, befreite die Lösung vom Toluol und unterwarf den Rückstand einer Vakuumdestillation über eine 10 cm Vigreux-Kolonne. Man erhielt 390 g (68 % der Theorie) 2-(o-Methylbenzyl)acetessigsäureethylester mit einem Siedepunkt von 100 bis 105 °C bei 0,01 Torr.

Beispiel 2

2-Chlor-2-(o-methylbenzyl)acetessigsäureethylester

Zu 511,0 g (2,2 mol) 2-(o-Methylbenzyl)acetessigsäureethylester tropfte man unter Rühren innerhalb von 2 h 325 g (2,4 mol) Sulfurylchlorid zu. Anschließend erwärmte man noch 4 h auf 50 bis 55 °C. Das Rohprodukt wurde durch Vakuumdestillation ohne Kolonne gereinigt. Man erhielt 547 g (92 % der Theorie) 2-Chlor-2-(o-methylbenzyl)acetessigsäureethylester mit einem Siedepunkt von 120 °C bei 0,01 Torr.

Beispiel 3

o-Methylzimtsäure

Zu 4,85 mol 25 %-iger, wäßriger NaOH-Lösung wurden innerhalb von einer Stunde 292 g (1,08 mol) 2-Chlor-2-(o-methylbenzyl)acetessigsäureethylester zugetropft. Anschließend rührte man 6 Stunden unter Rückfluß nach. Die Reaktionslösung wurde zweimal mit Toluol ausgeschüttelt, anschließend mit halbkonzentrierter Salzsäure angesäuert und der ausgefallene Niederschlag abgesaugt. Man wusch mit Wasser und Toluol nach und trocknete im Trockenschrank. Man erhielt 157 g (90 % der Theorie) o-Methylzimtsäure mit einem Schmelzpunkt von 176 bis 177 °C.

Beispiel 4

o-Methylzimtsäurephenylethylester

Eine Lösung aus 324,0 g (2,00 mol) o-Methylzimtäure, 428,0 g (3,5 mol) 2-Phenylethanol, 10,0 g (0,058 mol) p-Toluolsulfonsäure und 1200 ml Toluol wurde am Wasserabscheider unter Rückfluß erhitzt. Nach 15 Stunden hatte sich die berechnete Menge Wasser abgeschieden. Die Reaktionslösung wurde zuerst mit Wasser, dann mit gesättigter Natriumhydrogencarbonatlösung gewaschen. Das vereinigte Waschwasser wurde einmal ausgeethert und die Etherphase mit der organischen Phase vereinigt. Die vereinigten, organischen Phasen fraktionierte man über eine 10 cm Vigreux-Kolonne. Bei Normaldruck und im Wasserstrahlvakuum wurden die Lösungsmittel abgetrennt. Man wechselte die Vigreux-Kolonne gegen einen Kolonnenkopf aus und destillierte das Produkt bei 0,01 Torr Sdp. 154 bis 155°C/0,01 Torr. Man erhielt 394,0 g o-Methylzimtsäurephenylethylester mit einem Schmelzpunkt von 52 bis 53 °C.

Beispiel 5

Blumige Parfümbase mit Gardenia-Charakter

## Gewichtsanteile

| | |
|---|---|
| 20 | ß-Methylnaphthylketon crist. |
| 15 | Diphenyloxid |
| 20 | Zimtalkohol |
| 80 | Amylsalicylat iso |
| 10 | Styrolylacetat |
| 10 | Acetophenon 10 % i. DPG |
| 20 | Anisaldehyd |
| 50 | Dimethylbenzylcarbinol |
| 15 | Dimethylbenzylcarbinylacetat |
| 10 | Methylacetophenon 10 % i. DPG |
| 50 | Phenylethylalkohol |
| 60 | Jonon rein |
| 15 | Aldehyd C 11 20 % i. DPG |
| 20 | Aldehyd C 11 en 20 % i. DPG |
| 15 | Aldehyd C 12 MNA 20 % i. DPG |
| 80 | Benzylacetat |
| 110 | alpha-Amylzimtaldehyd |
| 5 | Isoeugenol |
| 10 | Eugenol |
| 30 | Canangaöl |
| 100 | Rose synthetisch |
| 55 | 8-Cyclohexadecenon |
| 100 | 2,2-Dimethyl-3-(m-methylphenyl)propanol |
| 100 | o-Methylzimtsäurephenylethylester |

Summe: 1000

Beispiel 6

Blumige Parfümbase mit Fliedercharakter

4

Gewichtsanteile

| 280 | Terpineol |
|---|---|
| 160 | 2,2-Dimethyl-3-(m-methylphenyl)propanol |
| 80 | Phenylethylalkohol |
| 60 | Benzylacetat |
| 5 | Anisaldehyd |
| 5 | Phenylacetaldehyddimethylacetal |
| 5 | Isoeugenol |
| 10 | Zimtalkohol |
| 20 | Linalool |
| 80 | Heliotropin |
| 20 | Indol 10 % i. DPG |
| 70 | Benzylalkohol |
| 40 | Ylang Eco Ess. synthetisch |
| 20 | Cyclamenaldehyd |
| 5 | Aldehyd C 12 MNA 5 % i. DPG |
| 40 | 8-Cyclohexadecenon |
| 100 | o-Methylzimtsäurephenylethylester |

Summe 1000

**Patentansprüche**

1. o-Methylzimtsäurephenylethylester.

2. Verfahren zur Herstellung von o-Methylzimtsäurephenylethylester, dadurch gekennzeichnet, daß Acetessigester in einer phasentransferkatalysierten Alkylierung mit o-Methylbenzylchlorid umgesetzt wird.

3. Verfahren nach Anspruch 2, dadurch gekennzeichnet, daß als weitere Verfahrensschritte eine Halogenierung des erhaltenen 2-(o-Methylbenzyl)acetessigsäureethylesters, eine Umsetzung des erhaltenen 2-Halogen-2-(o-methylbenzyl)acetessigsäureethylesters mit Base und eine Veresterungder erhaltenen o-Methylzimtsäure mit 2-Phenylethanol durchgeführt werden.

4. Verwendung von o-Methylzimtsäurephenylethylester als Riechstoff.

**Claims**

1. Phenylethyl o-methylcinnamate.

2. Process for the preparation of phenylethyl o-methylcinnamate, characterised in that ethyl acetoacetate is alkylated with o-methylbenzyl chloride using a phase transfer catalyst.

3. Process according to Claim 2, characterised in that halogenation of the resulting ethyl 2-(o-methylbenzyl)-acetoacetate, reaction of the resulting ethyl 2-halo-2-(o-methylbenzyl)-acetoacetate with a base and esterification of the resulting o-methylcinnamic acid with 2-phenylethanol are carried out as further process steps.

4. Use of phenylethyl o-methylcinnamate as a fragrance material.


**Revendications**

1. o-méthylcinnamate de phényléthyle.

2. Procédé de préparation de l'o-méthylcinnamate de phényléthyle, caractérisé en ce qu'on fait réagir un ester de l'acide acétoacétique dans le cadre d'une alkylation catalysée par un catalyseur de transfert de phase avec du chlorure d'o-méthylbenzyle.

3. Procédé selon la revendication 2, caractérisé en ce qu'on met en oeuvre en tant qu'étapes supplémentaires du procédé une halogénation de o-méthylbenzyl-2 acétoacétate d'éthyle obtenu, une réaction de l'halogéno-2 o-méthylbenzyl-2 acétoacétate d'éthyle obtenu avec une base, et une estérification de l'acide o-méthylcinnamique obtenu avec du 2-phényléthanol.

4. Utilisation de l'o-méthylcinnamate de phényléthyle en tant que matière odorante.